**Europäisches Patentamt**

**European Patent Office**

**Office européen des brevets**

(11) Numéro de publication: **0 094 367**
**A2**

## (12) DEMANDE DE BREVET EUROPEEN

(21) Numéro de dépôt: **83870045.8**

(22) Date de dépôt: **29.04.83**

(51) Int. Cl.³: **A 61 B 5/05**, A 61 B 5/10,
G 01 R 27/02

(30) Priorité: **07.05.82 BE 208035**

(43) Date de publication de la demande: **16.11.83**
**Bulletin 83/46**

(71) Demandeur: **Niessen, Pierre, rue de Battice 4,**
**B-4822 Petit-Rechain (BE)**
Demandeur: **Toupy, Jean-Léon, rue de Dison 175,**
**B-4822 Petit-Rechain (BE)**

(72) Inventeur: **Niessen, Pierre, rue de Battice 4,**
**B-4822 Petit-Rechain (BE)**
Inventeur: **Toupy, Jean-Léon, rue de Dison 175,**
**B-4822 Petit-Rechain (BE)**

(84) Etats contractants désignés: **AT CH DE FR GB IT LI LU**
**NL SE**

(74) Mandataire: **Vanderperre, Robert et al, Bureau VANDER**
**HAEGHEN 63 Avenue de la Toison d'Or,**
**B-1060 Bruxelles (BE)**

(54) **Procédé et appareil pour déterminer l'état d'une zone de la surface de la peau.**

㉗ A l'aide d'une sonde à deux électrodes (1, 2) on connecte la zone de surface de peau considérée entre deux points d'un circuit électronique comprenant un générateur de tension (10) organisé pour produire une tension électrique proportionnelle à la conductibilité électrique de la peau dans la zone considérée. Cette tension est utilisée pour attaquer un dispositif de mesure ou d'indication (30) organisé pour donner une indication variant suivant une relation donnée avec l'amplitude de la tension.

EP 0 094 367 A2

## Procédé et appareil pour déterminer l'état d'une zone de la surface de la peau

La présente invention concerne un procédé et un appareil pour déterminer de façon rapide et précise l'état d'une zone quelconque de la peau.

En dermatologie et cosmétologie il importe de pouvoir déterminer la nature et l'état d'une zone de la peau à traiter ou à soigner. On connaît différents procédés pour déterminer la nature de la surface de la peau ou d'une zone de la peau : sèche, normale, mixte, grasse. Les procédés connus consistent soit à examiner l'aspect de la peau à l'aide d'une loupe grossissante, soit à appliquer sur la peau un papier sensible qui absorbe ou retient les matières grasses sécrétées ou encore qui réagit à l'acidité de la peau. Ces procédés s'avèrent relativement empiriques et peu précis. Ils ne permettent en tout cas guère de déterminer avec précision l'éfficacité d'un produit pour la peau, ou la rapidité de son effet pour la persistance de l'effet d'un produit dermatologique ou cosmétique.

Connaître simplement le type de peau est insuffisant car cela ne permet en aucune façon de vérifier l'efficacité réelle d'un produit ou d'un traitement sur l'état de la peau. Prenons par exemple le cas du dos de la main qui est généralement sec et d'une crème adoucissante pour les mains. Lorsque l'on applique la crème adoucis-

sante sur le dos de la main, la crème pénètre progressivement dans la peau et son effet réel ne peut être déterminé qu'un constatant non seulement la modification temporaire de l'état de la peau mais également l'importance relative de cette modification temporaire d'état et la durée de l'amélioration due à ce produit. Il est évident qu'au moment de l'application de la crème, la peau a l'aspect gras mais c'est un certain temps après l'application de la crème que l'on doit vérifier son effet. Il arrive fréquemment que la peau redevienne rapidement sèche après l'application d'un produit et la simple constatation de l'effet immédiat est dès lors insuffisante pour apprécier la valeur réelle ou l'efficacité du produit.

Le problème réside donc dans le fait de pouvoir constater rapidement et avec une précision satisfaisante l'effet d'un produit pour la peau et la durée de cet effet car de cette durée découlera la fréquence d'application du produit pour que soit obtenu un résultat réel quant à l'amélioration de l'état de la peau. Une crème qui se triyve absorbée en quelques minutes seulement et laisse donc la peau sèche dès que ces quelques minutes sont écoulées ne peut produire d'amélioration véritable qu'à la condition de réappliquer cette crème de façon continue. Par contre, une crème dont l'effet persiste pendant plusieurs heures nécessite une moindre fréquence d'application pour améliorer l'état de la peau.

Ce problème est résolu selon l'invention par un procédé de détermination de l'état d'une zone de la surface de la peau selon lequel on connecte la zone de surface de peau considérée entre deux points d'un circuit électronique ,on détecte la chute de tension électrique sur la zone de peau considérée,on crée une tension électrique proportionnelle à la conductibilité électrique de la peau dans la zone considérée et on utilise ladite tension électrique pour attaquer un dispositif de mesure ou d'indication approprié, pour donner une indication variant suivant une relation donnée avec ladite tension.

Ce procédé est mis en oeuvre dans un appareil qui comprend une sonde comportant deux électrodes conductrices de l'électricité, ces électrodes étant écartées l'une de l'autre et destinées à être mises en contact avec la zone de peau considérée ; un générateur de tension électrique connecté aux deux électrodes, ce générateur étant organisé pour produire une tension électrique proportionnelle à la conductibilité électrique de la peau connectée entre les deux électrodes précitées; un dispositif indicateur connecté à la sortie du générateur de tension électrique pour donner une indication variant suivant une relation donnée avec l'amplitude de la tension produite par le générateur. Avantageusement, l'appareil comprend encore un dispositif de contrôle connecté pour comparer la tension électrique du générateur de tension avec des tensions de seuils prédéterminées et produire des signaux de commande pour des dispositifs de commutation connectés pour produire des signaux de dépassement de limites de la zone de mesure.

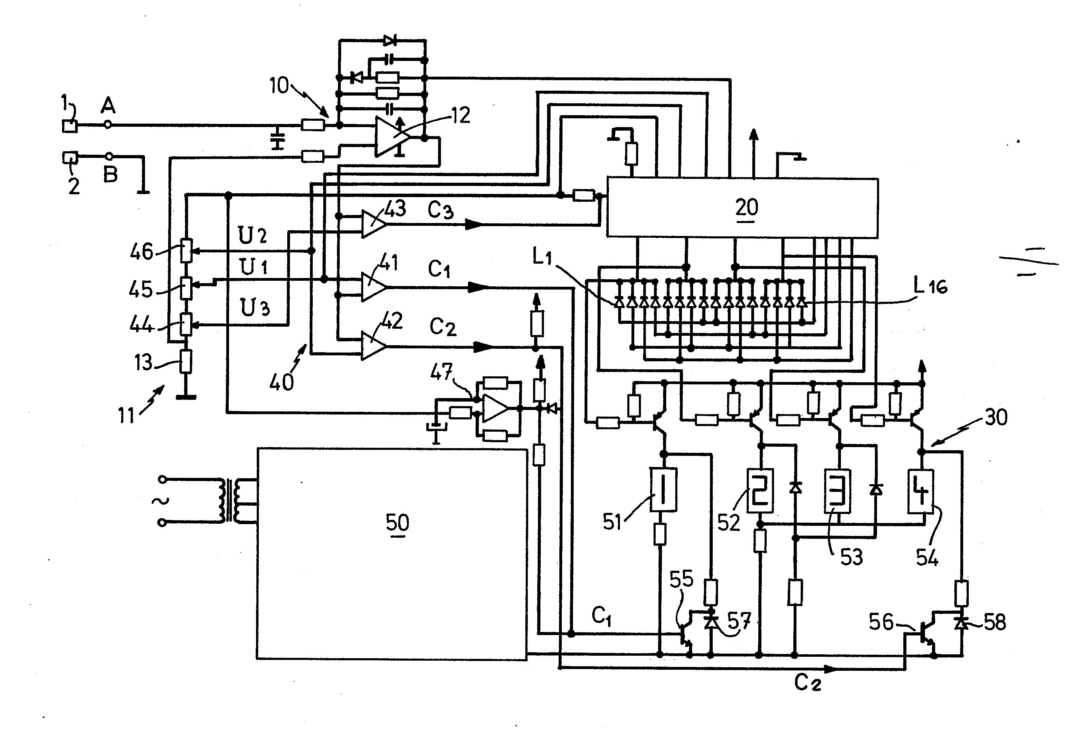

L'invention est exposée plus en détail dans ce qui suit avec référence au dessin ci-annexé qui illustre un exemple de mode d'exécution.

La solution proposée par la présente invention consiste à utiliser la conductibilité électrique de la peau qui varie selon l'état de celle-ci. A l'aide d'une sonde de mesure à deux électrodes, on connecte la zone de surface de peau à examiner entre deux points d'un circuit électronique mis sous basse tension en sorte d'insérer dans ce circuit la résistance électrique que présente la peau. La chute de tension électrique sur la zone de surface de peau entre les deux électrodes est détectée pour produire un signal de mesure proportionnel. Celui-ci varie linéairement avec la résistance électrique de la peau,c'est-à-dire selon l'état effectif de la peau à l'endroit examiné. Le signal de mesure produit est ensuite utilisé pour attaquer un dispositif de mesure ou d'indication approprié. La zone de mesure couvrant les quatre types de peau (peau sèche, peau normale, peau mixte, peau grasse) s'étend linéairement sur une gamme de valeurs de résistance variant typiquement d'environ 4,4 mégohms à environ 12 mégohms. En observant la variation de la tension de mesure produite il est ainsi possible de suivre au moment même l'évolution et donc de suivre au fur et à mesure qu'elle se déroule l'évolution de l'effet d'un produit appliqué sur la peau, à savoir : l'ampleur de la variation d'état qu'il produit sur la peau et la persistance de son effet.

La figure ci-annexée est un schéma du circuit d'une forme de réalisation avantageuse d'un appareil réalisant le procédé de mesure décrit ci-dessus.

Les électrodes 1 et 2 de la sonde de mesure sont par exemple des broches de laiton chromé à bout arrondi,fixées dans un corps en matière plastique avec un écartement de quelque 12 mm par exemple. Les électrodes sont connectées au moyen d'un câble blindé aux bornes A et B du circuit électronique. Celui-ci comprend essentiellement un générateur de tension 10, un convertisseur analogique/numérique 20, un dispositif d'affichage 30 et un dispositif de contrôle réglable 40. Le générateur de tension 10 est constitué d'un circuit d'entrée 11 comprenant une résistance 13 et la résistance connectée entre les bornes A et B, et d'un amplificateur opérationnel 12. Celui-ci produit une tension continue proportionnelle au courant traversant l'impédance connectée entre les bornes A et B c'est-à-dire entre les électrodes 1 et 2 de la sonde.

Le convertisseur analogique/numérique 20 consiste par exemple en une unité intégrée UAA 170 de la firme Siemens servant à la commande d'un dispositif d'affichage numérique à diodes LED par multiplexage. Le convertisseur 20 est organisé pour convertir les tensions de sortie de l'amplificateur 12 en $\underline{N}$ signaux numériques répartis linéairement dans la gamme de mesures couvrant les indications correspondant aux quatre types de peau. Cette gamme de mesure qui s'étend globalement entre des valeurs qui correspondent à des résistances d'environ 4,4 mégohms et d'environ 12 mégohms, est avantageusement divisée ici en seize portions consécutives : la zone correspondant à chaque type de peau est donc divisée en quatre portions visualisées chacune par un afficheur lumineux Li. Se trouve ainsi chaque fois illuminé l'afficheur qui représente la valeur numérique correspondant à la tension électrique proportionnelle à la conductibilité de la zone de peau connectée entre les électrodes de la sonde et cette indication visuelle indique dans quelle partie de la zone correspondant à un type de peau est située la mesure représentative de la peau examinée. Si l'on désigne par L1, L2, L3, L4, L5,..L16 les seize indications visuelles couvrant la gamme de mesure dans l'exemple décrit, ces indications correspondent aux différents types de peau comme suit : peau sèche (zone 1): L1.... L4

peau normale (zone 2): L5.... L8

peau mixte   (zone 3): L9.... L12

peau grasse  (zone 4): L13... L16

Une mesure L4, par exemple, indique une peau sèche, mais presque normale et une indication L5, par exemple, indique une peau normale, mais qui est presque une peau sèche. L'indication linéaire fournie conformément à l'invention permet donc de situer de façon précise l'état d'une zone de peau examinée dans la gamme de mesure des types de peau. Reprenant l'exemple décrit plus haut, d'une crème adoucissante pour les mains, l'invention permet de suivre l'effet du produit appliqué sur le dos de la main et de constater immédiatement et avec précision l'importance de l'effet de la crème sur les mains (afficheur d'indice maximum illuminé) et l'évolution de l'effet sur les mains (illumination successive d'afficheurs d'indice inférieur). La cadence de rétrogradation de l'illumination des afficheurs est représentative de la persistance de l'effet du produit ou d'un traitement.

Dans l'exemple illustré, les afficheurs L1 - L16 sont doublés par quatre voyants numériques de zone 51-54. Chacun de ces voyants se trouve illuminé en même temps que l'un quelconque des afficheurs $L_i$ associés à la zone de mesure correspondante.

L'appareil se complète d'un dispositif de contrôle réglable destiné à fixer les limites de fonctionnement de l'appareil. Dans l'exemple de la figure ci-annexée, le dispositif de contrôle comprend essentiellement trois comparateurs 41-43. Le premier comparateur 41 est connecté pour comparer la tension de mesure à la sortie de l'amplificateur 10 avec une première tension de seuil U1 réglée sur le potentiomètre 45 inséré dans le circuit d'entrée. Cette tension de seuil U1 est choisie pour correspondre à une résistance entre les électrodes de la sonde égale à la limite inférieure de la gamme de mesure, par exemple 4,4 mégohms. Lorsque la tension de mesure est inférieure à ce premier seuil U1, le comparateur 41 produit un signal de commande C1 qui se trouve appliqué comme signal de validation à la base d'un transistor de commutation 55. Celui-ci est connecté aux bornes d'une diode 57 shuntant le voyant numérique 51 de la zone 1 de mesure. Un signal d'un oscillateur 47 est alors appliqué au transistor 55 afin de faire clignoter le voyant 51 en sorte d'indiquer visuellement que la limite inférieure de la zone 1 est dépassée, c'est-à-dire d'indiquer que la zone de peau examinée a un état situé hors de la gamme de mesure (peau extrêmement sèche).

Le comparateur 42 est connecté pour comparer la tension de mesure avec une deuxième tension de seuil U2 réglée sur le potentiomètre 46 inséré dans le circuit d'entrée. Cette tension de seuil U2 est choisie pour correspondre à une résistance entre les électrodes de la sonde égale à la limite supérieure de la gamme de mesure, par exemple 12 mégohms. Lorsque la tension de mesure est supérieure à ce deuxième seuil U2, le comparateur 42 produit un signal de commande C2 qui se trouve appliqué comme signal de validation à la base d'un transistor de commutation 56 connecté aux bornes d'une diode 58 qui shunte le voyant numérique 54 de la zône 4 de mesure. Le signal de l'oscillateur 47 est alors appliqué au transistor 56 afin de faire clignoter le voyant 54 pour indiquer visuellement le dépassement de la limite supérieure de la zone 4, c'est-à-dire pour indiquer que la zone de peau examinée a un état situé hors de la gamme de mesure (peau extrêmement grasse).

Le troisième comparateur 43 est connecté pour comparer la tension de mesure avec une troisième tension de seuil U3 réglée sur le potentiomètre 44 inséré dans le circuit d'entrée. Cette tension de seuil U3 est choisie pour correspondre à une résistance entre les électrodes de la sonde de mesure égale à une valeur relativement élevée,

0094367

par exemple 1000 mégohms. Lorsque la tension de mesure dépasse ce troisième seuil U3, le comparateur 43 produit un signal de commande C3 pour inhiber l'illumination du dispositif d'affichage de manière à protéger les circuits contre des signaux exagérés non significatifs.

## R E V E N D I C A T I O N S

1.   Procédé pour déterminer l'état d'une zone de la surface de la peau, caractérisé en ce qu'on connecte la zone de surface de peau considérée entre deux points d'un circuit électronique, on détecte la chute de tension électrique sur la zone de peau considérée, on crée une tension électrique proportionnelle à la conductibilité électrique de la peau dans la zone considérée , et on utilise ladite tension électrique pour attaquer un dispositif de mesure ou d'indication approprié  pour donner une indication variant suivant une relation donnée avec ladite tension.

2.   Appareil pour déterminer l'état d'une zone de la surface de la peau, caractérisé  par une sonde comportant deux électrodes (1,2) conductrices de l'électricité, ces électrodes étant écartées l'une de l'autre et destinées à être mises en contact avec la zone de peau considérée;
un générateur de tension électrique (10) connecté aux deux électrodes, ce générateur étant organisé pour produire une tension électrique proportionnelle à la conductibilité électrique de la peau connectée entre les deux électrodes précitées;
un dispositif indicateur (30) connecté à la sortie dudit générateur de tension électrique pour donner une indication variant suivant une relation donnée avec l'amplitude de la tension produite par ledit générateur.

3.   Appareil selon la revendication 2, caractérisé par un dispositif de contrôle (40) connecté pour comparer la tension électrique du générateur de tension (10) avec une première tension de seuil prédéterminée (U1) et produire un premier signal de commande (C1), un premier dispositif de commutation (55) connecté pour répondre audit premier signal de  commande  et produire un premier signal de dépassement de limite.

4. Appareil selon la revendication 3, caractérisé en ce que le dispositif de contrôle réglable (40) comprend des moyens (42) pour comparer la tension électrique du générateur de tension (10) avec une deuxième tension de seuil prédéterminée (U2) et produire un deuxième signal de commande (C2), et un deuxième dispositif de commutation (56) connecté pour répondre audit deuxième signal de commande et produire un deuxième signal de dépassement de limite.

5. Appareil selon l'une quelconque des revendications 3 et 4, caractérisé en ce que le dispositif de contrôle (40) comprend des moyens (43) pour comparer la tension électrique du générateur de tension (10) avec une troisième tension de seuil prédéterminée (U3) et produire un troisième signal de commande (C3), et un troisième dispositif de commutation connecté pour répondre audit troisième signal de commande et produire un signal pour couper de l'indication de mesure proportionnelle à la tension électrique du générateur de tension.

6. Appareil selon l'une quelconque des revendications 2 à 5, caractérisé par un convertisseur analogique/ numérique (20) connecté entre le générateur de tension (10) et le dispositif indicateur (30) pour convertir les amplitudes de la tension électrique du générateur de tension, situées dans une gamme de mesure prédéterminée, en un nombre prédéterminé quelconque de signaux de mesure numériques.

7. Appareil selon la revendication 6, caractérisé en ce que le dispositif indicateur (30) comprend des premiers moyens de visualisation (51-54) connectés pour afficher une indication numérique distincte pour chaque zone correspondant à un type de peau; et un nombre N de seconds moyens de visualisation(L1,...L16) connectés pour afficher N indications réparties linéairement
dans la gamme de mesure couverte par lesdits premiers
moyens de visualisation(51, 52, 53, 54).

0094367

Fig. 1/1